# EUROPEAN PATENT SPECIFICATION

(11) **EP 2 913 004 B1**
(45) Date of publication and mention of the grant of the patent: **14.09.2016**
(21) Application number: 15156645.2
(22) Date of filing: 26.02.2015
(51) Int. Cl.: A61B 5/15

(54) **Handheld needle guard for use in cannulation**
Tragbare Nadelführung zur Verwendung in einer Kanüle
Protection d'aiguille portative destinée à être utilisée dans la canulation

(30) Priority: 28.02.2014 SE 1450228
(43) Date of publication of application: 02.09.2015
(73) Proprietor: Wästrahult Innovation AB, 352 63 Växjö (SE)
(72) Inventor: Barrdahl, Fredrik, 352 63 Vaxjö (SE); Andersson, Jan-Olof, 352 63 Växjö (SE)
(74) Representative: Awapatent AB

(56) References cited:
- FR-A1- 2 954 085
- US-A- 5 674 227
- US-A- 5 690 646
- US-A1- 2001 054 429

## Description

### Technical field

The present invention relates to a handheld needle guard to be used in cannulation of the umbilical cord of neonates.

### Background art

When a child is born, two samples have to be taken from two different blood vessels of the umbilical cord within a space of 30 to 60 seconds after birth, i.e. before the umbilical cord is cut. This is done with the aid of a cannula, so as to be able to quickly ascertain how the child is doing and how it has taken in oxygen during the actual delivery. The situation may often be stressful for the medical personnel in view of the time constraints and the fact that the mother and the newborn child may make unexpected movements during the sampling procedure. The umbilical cord is covered with several different fluids, which makes it wet, slippery and difficult to handle, which means there is a considerable risk that the person carrying out the sampling procedure will be pricked by the needle. The person carrying out the sampling procedure is then exposed to a risk of being infected by various diseases that are transmissible via blood. In May 2013, an EU directive (2010/32/EU) was also introduced that sets out the requirements for the health sector to protect its personnel from needlestick injuries and sharps injuries.

US 2001/0054429 discloses a handheld needle guard for taking samples from the umbilical cord of neonates. The needle guard consists of a sleeve that has been produced from a hollow tube, the latter having been divided in the longitudinal direction, and is placed in the palm of the hand of the person taking the sample, such that it extends in the longitudinal direction towards the placenta. The umbilical cord is then laid in the sleeve and is secured by placing the thumb over the umbilical cord and the sleeve. A disadvantage of the guard according to US 2001/0054429 is that the whole hand is used and cannot help in the actual sampling. The needle guard is also placed in the palm of the hand, which means it is easy to accidentally insert the cannula into the hand instead of into the umbilical cord.

### Disclosure of the invention

It is an object of the invention to make available a handheld needle guard which makes it easy to take samples from an umbilical cord without any risk of needlestick injuries or sharps injuries. According to the present invention, this aim is achieved by a handheld needle guard for use in cannulation of the umbilical cord of neonates, said needle guard having an umbilical cord channel which, along its length, is composed of a securing portion and, following on from the latter, a cannulation portion, said securing portion having a grip part, which protrudes from the underside of the umbilical cord channel.

The handheld needle guard is thus composed of two portions with different functions and also comprises a grip part that protrudes from underneath the securing portion of the needle guard such that the cannulation portion is not placed directly in the palm of the hand.

An advantage of this needle guard is that it does not take up the whole hand holding the needle guard, and instead it allows parts of the hand to be used to help in the cannulation. A further advantage is that the cannulation portion itself is at least partly located outside the hand or the palm of the hand, which reduces the risk of needlestick injuries. Moreover, the needle guard is simple, inexpensive to produce, easy to clean and user-friendly.

According to one embodiment, the grip part protrudes substantially perpendicularly in relation to the longitudinal direction of the umbilical cord channel. An advantage of this embodiment is that it allows the grip part to be held firmly between two fingers, for example the middle finger and index finger. The other fingers of the hand are therefore free during the cannulation.

According to one embodiment, the grip part is designed to be locked firmly between two fingers, preferably between the index finger and the middle finger. By arranging the grip part in this way, the greater part of the hand is placed under the actual umbilical cord channel, especially under the securing portion, and there is less risk of pricking the hand, and it is also possible to use all the fingers to help in the sampling procedure. It should also be possible to use the needle guard to insert a peripheral venous catheter (PVC) for delivery of medication if the child is unwell directly after delivery. When inserting such a venous catheter into the umbilical cord, the fingers are needed to help attach the catheter to the umbilical cord while at the same time the needle guard has to be held firmly between two fingers.

Said umbilical cord channel can be arched, U-shaped or polygonal. In this way, a channel with walls is formed in which the umbilical cord is placed and is held in position in part by the walls of the channel, in which case a good overview of the sampling from the umbilical cord is obtained since the latter entails three blood vessels, but a sample is to be collected from only two of them. Moreover, it is easy to change position and rotate the umbilical cord to the desired place in the umbilical cord channel.

Said cannulation portion of the umbilical cord channel is expediently provided at least in part with grooves, which help hold the umbilical cord in place in the needle guard and provide an indication to the user of where the cannulation is to take place. This portion is placed outside the hand and in this way the risk of needlestick injuries is eliminated.

Said securing portion is expediently provided with a notch for gripping by the thumb, which means that the umbilical cord channel in this portion is partially without walls and forms a surface for holding the umbilical cord against.

In one embodiment, the grip part is provided with flutes in order to help lock it firmly between two fingers. An advantage of these flutes is that they give a surface that is grooved and easier to grip with the fingers.

In one embodiment, the cannulation portion is provided with a direction indicator, which points in a direction away from the securing portion. The direction indicator shows how the needle guard is to be placed in the hand, i.e. with the cannulation portion directed away from the hand so that the cannulation portion lies outside the hand. This helps in stressful situations.

In one embodiment, the grip part is configured as a rectangle, which provides a natural grip for securing the grip part between two fingers. In this way, the needle guard can be used equally well by right-handed and left-handed persons.

Said umbilical cord channel expediently has a length of between 6 and 20 cm. This results in a needle guard that is easy to handle and is not hard to clasp between two fingers. The cannulation portion expediently has a length of 3-8 cm. The securing portion expediently has a length of 3-12 cm. In this way, sufficient space is provided for the cannulation, and also sufficient space for the hand that is holding the needle guard by the securing portion, so that this hand does not impede the cannulation.

Said umbilical cord channel expediently has an internal width of 15-35 mm, which means that it can be used for umbilical cords of different thicknesses, i.e. one guard can be used for all thicknesses of umbilical cords, so that it is not necessary to choose a needle guard according to the size of the umbilical cord and thereby increase the time from birth to cannulation.

The front part of the cannulation portion can be designed as a concavity, for example an arc, concave ring or other type of concavity. The material edge on the front part of the cannulation portion can be cut in such a way that the edge forms a bevel. The cannulation portion can be designed with one of these features or a combination thereof, since both these features help to prevent the umbilical cord from sliding out of the needle guard.

The needle guard can be made of metal or plastic but is preferably made of plastic since this is a less expensive product that can be easily cleaned or discarded if not used again.

Moreover, the needle guard will also be able to be used as an aid in sampling procedures in developing countries since it is cheap to produce, easy to clean and easy to use, the direction indicator in the cannulation portion showing the user in which direction the needle guard is to be placed.

According to one embodiment, the needle guard has left-right symmetry, seen along the longitudinal direction of the umbilical cord channel. An advantage of this is that the needle guard can be held in either hand and can be used both by right-handed and also by left-handed individuals.

### Brief description of the drawings

Preferred embodiments of the invention are described in more detail below with reference to the accompanying drawings, in which:
Fig. 1 shows schematically a needle guard according to the invention seen obliquely from the front.
Fig. 2 shows schematically a needle guard according to the invention seen from the side.
Fig. 3 shows schematically a needle guard according to the invention seen from above.
Fig. 4 shows schematically a needle guard according to the invention seen from underneath.
Figs 5 and 6 show schematically the positioning of the needle guard in a hand, and how a sample is taken from the umbilical cord.

### Detailed description of the preferred embodiments

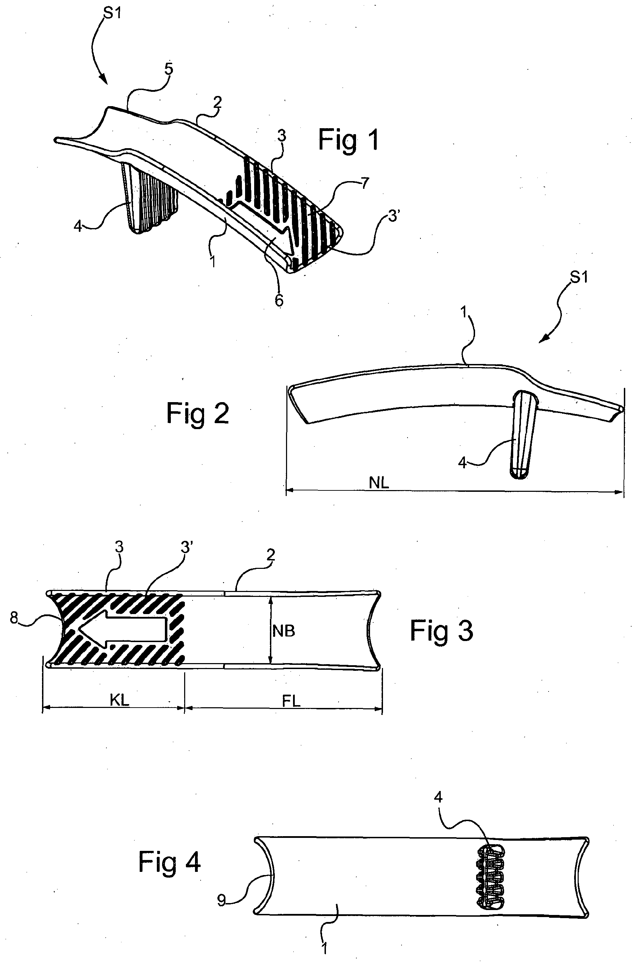

Figs 1-4 show a handheld needle guard S1 according to the present invention, seen from different angles. The needle guard S1 consists of an elongate umbilical cord channel 1, which is composed of a securing portion 2 and a cannulation portion 3, which are arranged in succession along the longitudinal direction of the umbilical cord channel 1. A grip part 4 is arranged on the underside of the securing portion 2. The grip part 4 extends substantially perpendicularly downward in relation to the longitudinal direction of the umbilical cord channel 1.

The securing portion 2 is provided with a notch 5 for gripping by the thumb, so that someone taking a sample can easily hold an umbilical cord pressed against the securing portion 2.

The cannulation portion 3 is further provided with grooves 3', which visually indicate within which needle zone 7 the actual sampling/injection is to take place. The cannulation portion 3 can also be provided with a direction indicator 6, for example in the form of an arrow showing in which direction the needle guard S1 is to be held.

The front part 8 of the cannulation portion 3 can be configured as a concavity, for example an arc, concave ring or other type of concavity. The material edge 9 of this concavity, as shown in Fig. 4, can be cut in such a way that the edge forms a bevel, so as to prevent the umbilical cord from sliding out of the umbilical cord channel 1.

As will be seen from Fig. 1, the grip part 4 can be provided with flutes and can have the shape of a rectangle in order to provide a natural grip and good locking between two fingers.

The length NL of the umbilical cord channel 1, as shown in Fig. 2, can expediently vary between 6 and 20 cm, which gives a needle guard that is easy to handle and that is not too hard to clasp firmly between two fingers.

The length KL of the cannulation portion 3 can expediently be between 3 and 8 cm, and the length FL of the securing portion 2 can expediently be between 3 and 12 cm, as is shown in Fig. 3, which gives sufficient space for cannulation and sufficient room for the hand holding the needle guard by the securing portion 2, so that it does not get in the way of the cannulation.

The umbilical cord channel 1 expediently has an internal width NB of 15-35 mm, as shown in Fig. 3, which means that a needle guard S1 can be used for different thicknesses of umbilical cords.

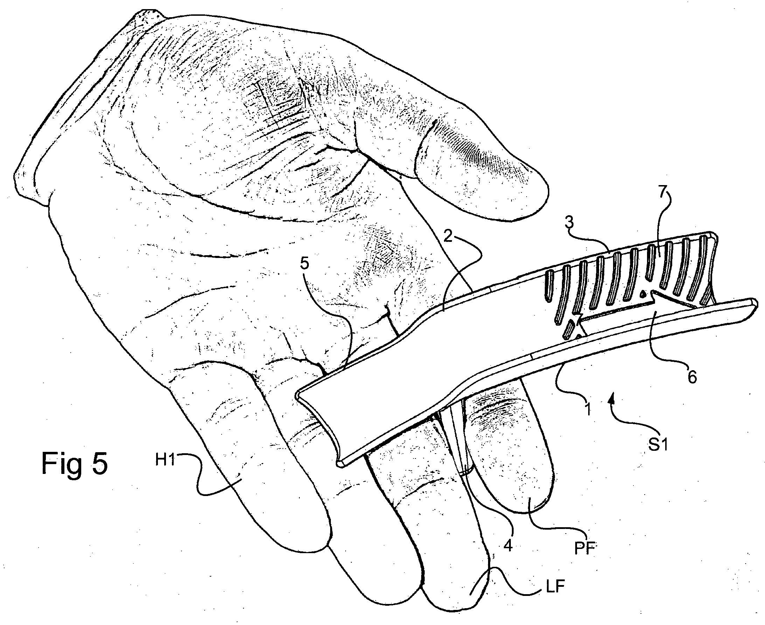

Fig. 5 shows the placing of the needle guard S1 in a hand H1 of the person who is to take a sample from an umbilical cord or is to insert a peripheral venous catheter (PVC) into the umbilical cord. When the child is born, the needle guard S1 is placed in the hand with the cannulation portion 3 such that the marked needle zone 7 and the direction indicator 6 on the cannulation portion 3 are directed away from the hand H1. The grip part 4 of the securing portion 2 is clasped firmly between two fingers, for example between the index finger PF and the middle finger LF.

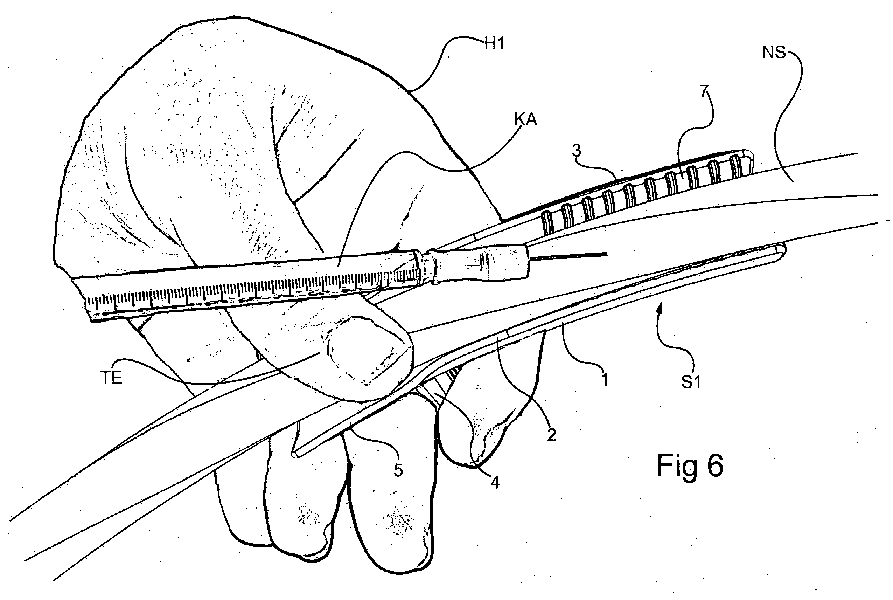

Fig. 6 shows how an umbilical cord NS is laid in the needle guard S1 and is held in place by the thumb TE of the hand H1 being laid over the umbilical cord NS in the notch 5 of the securing portion 2 and pressing the umbilical cord NS against the securing portion 2. The rest of the hand H1 in this way remains free to help take the sample or insert a PVC into the umbilical cord NS, and the greater part of the needle guard S1, and especially the cannulation portion 3, is arranged outside the hand H1, thus minimizing the risk of needlestick injuries. Fig. 6 also shows how a cannula KA, which is held in another hand (not shown), is inserted into the umbilical cord NS in the cannulation portion 3 of the umbilical cord channel 1, which portion is located outside the hand H1.

A person skilled in the art will appreciate that the described embodiment can be varied within the scope of the accompanying claims, e.g. for adaptation to different types of cannulation that can take place in an umbilical cord.

## Claims

1. A handheld cannulation needle guard for use in cannulation of the umbilical cord of neonates, **characterized by**
an umbilical cord channel (1) which, along its length (NL), comprises a securing portion (2) and, following on from the latter, a cannulation portion (3), said securing portion (2) having a grip part (4), which protrudes from the underside of the umbilical cord channel (1).

2. The handheld needle guard according to claim 1, wherein the grip part (4) protrudes substantially perpendicularly in relation to the longitudinal direction of the umbilical cord channel (1).

3. The handheld needle guard according to any of the preceding claims, wherein the grip part (4) is designed to be locked firmly between two fingers, preferably between the index finger and the middle finger.

4. The handheld needle guard according to any one of the preceding claims, wherein the umbilical cord channel (1) is arched, U-shaped or polygonal.

5. The handheld needle guard according to any one of the preceding claims, wherein the cannulation portion (3) of the umbilical cord channel (1) is provided at least in part with grooves.

6. The handheld needle guard according to any one of the preceding claims, wherein the securing portion (2) is provided with a notch (5) for gripping by the thumb.

7. The handheld needle guard according to any one of the preceding claims, wherein the grip part (4) is configured with flutes in order to help lock it firmly between two fingers, preferably between the index finger and the middle finger.

8. The handheld needle guard according to any one of the preceding claims, wherein the cannulation portion (3) is provided with a direction indicator (6), which points in a direction away from the securing portion (2).

9. The handheld needle guard according to any one of the preceding claims, wherein the grip part (4) is configured as a rectangle.

10. The handheld needle guard according to any one of the preceding claims, wherein the umbilical cord channel (1) has a length (NL) of between 6 and 20 cm.

11. The handheld needle guard according to any one of the preceding claims, wherein the cannulation portion (3) has a length (KL) of 3-8 cm, and the securing portion (2) has a length (FL) of 3-12 cm.

12. The handheld needle guard according to any one of the preceding claims, in which the umbilical cord channel (1) has an internal width (NB) of 15-35 mm.

## Patentansprüche

1. Tragbare Punktionsnadelführung zur Verwendung in der Punktion der Nabelschnur von Neugeborenen, **gekennzeichnet durch**
eine Nabelschnurrinne (1), die entlang ihrer Länge (NL) einen Befestigungsabschnitt (2) und, anschließend an den letztgenannten, einen Punktionsabschnitt (3) umfasst, wobei der Befestigungsabschnitt (2) einen Griffteil (4) aufweist, der von der Unterseite der Nabelschnurrinne (1) vorsteht.

2. Tragbare Nadelführung gemäß Anspruch 1, wobei das Griffteil (4) in Bezug auf die Längsrichtung der Nabelschnurrinne (1) im Wesentlichen senkrecht vorsteht.

3. Tragbare Nadelführung gemäß einem der vorhergehenden Ansprüche, wobei das Griffteil (4) ausgelegt ist, um fest zwischen zwei Fingern eingeschlossen zu werden, vorzugsweise zwischen dem Zeigefinger und dem Mittelfinger.

4. Tragbare Nadelführung gemäß einem der vorhergehenden Ansprüche, wobei die Nabelschnurrinne (1) gewölbt, U-förmig oder polygonal ist.

5. Tragbare Nadelführung gemäß einem der vorhergehenden Ansprüche, wobei der Punktionsabschnitt (3) der Nabelschnurrinne (1) mindestens teilweise mit Rillen versehen ist.

6. Tragbare Nadelführung gemäß einem der vorhergehenden Ansprüche, wobei der Befestigungsabschnitt (2) mit einer Einkerbung (5) zum Ergreifen durch den Daumen versehen ist.

7. Tragbare Nadelführung gemäß einem der vorhergehenden Ansprüche, wobei das Griffteil (4) mit Riefen konfiguriert ist, um zu helfen, es fest zwischen zwei Fingern einzuschließen, vorzugsweise zwischen dem Zeigefinger und dem Mittelfinger.

8. Tragbare Nadelführung gemäß einem der vorhergehenden Ansprüche, wobei der Punktionsabschnitt (3) mit einem Richtungsanzeiger (6) versehen ist, der in eine Richtung weg von dem Befestigungsabschnitt (2) weist.

9. Tragbare Nadelführung gemäß einem der vorhergehenden Ansprüche, wobei das Griffteil (4) als ein Rechteck ausgebildet ist.

10. Tragbare Nadelführung gemäß einem der vorhergehenden Ansprüche, wobei die Nabelschnurrinne (1) eine Länge (NL) zwischen 6 und 20 cm aufweist.

11. Tragbare Nadelführung gemäß einem der vorhergehenden Ansprüche, wobei der Punktionsabschnitt (3) eine Länge (KL) von 3 - 8 cm aufweist und der Befestigungsabschnitt (2) eine Länge (FL) von 3 - 12 cm aufweist.

12. Tragbare Nadelführung gemäß einem der vorhergehenden Ansprüche, wobei die Nabelschnurrinne (1) eine lichte Weite (NB) von 15 - 35 mm aufweist.

## Revendications

1. Protection d'aiguille de canulation portative destinée à être utilisée dans la canulation du cordon ombilical des nouveau-nés, **caractérisée par**:
un canal pour cordon ombilical (1) qui, sur sa longueur (NL), comprend une partie de fixation (2) et, après cette dernière, une partie de canulation (3), ladite partie de fixation (2) ayant une partie de préhension (4), qui dépasse depuis la face inférieure du canal pour cordon ombilical (1).

2. Protection d'aiguille portative selon la revendication 1, dans laquelle la partie de préhension (4) dépasse sensiblement perpendiculairement relativement au sens longitudinal du canal pour cordon ombilical (1).

3. Protection d'aiguille portative selon l'une quelconque des revendications précédentes, dans laquelle la partie de préhension (4) est conçue pour être fermement bloquée entre deux doigts, de préférence entre l'index et le majeur.

4. Protection d'aiguille portative selon l'une quelconque des revendications précédentes, dans laquelle le canal pour cordon ombilical (1) est en forme d'arc, de U ou polygonal.

5. Protection d'aiguille portative selon l'une quelconque des revendications précédentes, dans laquelle la partie de canulation (3) du canal pour cordon ombilical (1) est dotée au moins en partie de sillons.

6. Protection d'aiguille portative selon l'une quelconque des revendications précédentes, dans laquelle la partie de fixation (2) est dotée d'une encoche (5) pour être saisie par le pouce.

7. Protection d'aiguille portative selon l'une quelconque des revendications précédentes, dans laquelle la partie de préhension (4) est conçue avec des rainures de manière à aider son blocage ferme entre deux doigts, de préférence entre l'index et le majeur.

8. Protection d'aiguille portative selon l'une quelconque des revendications précédentes, dans laquelle la partie de canulation (3) est dotée d'un indicateur de direction (6), qui pointe dans une direction opposée à la partie de fixation (2).

9. Protection d'aiguille portative selon l'une quelconque des revendications précédentes, dans laquelle la partie de préhension (4) est conçue sous la forme d'un rectangle.

10. Protection d'aiguille portative selon l'une quelconque des revendications précédentes, dans laquelle le canal pour cordon ombilical (1) a une longueur (NL) comprise entre 6 et 20 cm.

11. Protection d'aiguille portative selon l'une quelconque des revendications précédentes, dans laquelle la partie de canulation (3) a une longueur (KL) de 3 à 8 cm, et la partie de fixation (2) a une longueur (FL) de 3 à 12 cm.

12. Protection d'aiguille portative selon l'une quelconque des revendications précédentes, dans laquelle le canal pour cordon ombilical (1) a une largeur interne (NB) de 15 à 35 mm.
